# EUROPEAN PATENT SPECIFICATION

(11) **EP 2 623 098 B1**
(45) Date of publication and mention of the grant of the patent: **07.09.2016**
(21) Application number: 12153878.9
(22) Date of filing: 03.02.2012
(51) Int. Cl.: A61K 31/00, A61K 31/216, A61P 35/00

(54) **Amide Amino Acid Esters of Nonsteroidal Anti-Inflammatory Drugs (NSAIDs) as Potent Inhibitors of Tumor Cell Growth**
Amid-Aminosäurenester von nichtsteroidalen entzündungshemmenden Arzneimitteln (NSAIDs) als potente Inhibitoren des Krebszellwachstums
Esters d'acide aminé d'amide de médicaments anti-inflammatoires non-stéroïdes (nsaid) en tant qu'inhibiteurs puissants de la croissance des cellules tumorales

(43) Date of publication of application: 07.08.2013
(73) Proprietor: King Saud University, 11421 Riyadh (SA)
(72) Inventor: Hassan, Tarek Aboul-Fadl Mohamed, 11451 Riyadh (SA); Al-Hamad, Suliman Saud, 11451 Riyadh (SA); Al-Obaid, Abdul-Rahman, 11451 Riyadh (SA); Piazza, Gary A., Mobile, AL Alabama 36604-1405 (US)
(74) Representative: Engelhard, Markus

(56) References cited:
- US-B1- 6 207 700
- Sunil V Mali ET AL: "Synthesis and evaluation of conjugates of non-steroidal anti-inflammatory drugs (NSAIDs) with valproic acid for antiproliferative properties and apoptosis induction", Journal of Pharmacy Research, 7 July 2011 (2011-07-07), pages 2136-2139, XP55029360, Retrieved from the Internet: URL:http://jpronline.info/index.php/jpr/ar ticle/view/8287/4231 [retrieved on 2012-06-08]
- PARCHA V ET AL: "Synthesis and pharmacological evaluation of naproxen and ibuprofen derivatives", JOURNAL OF THE INDIAN CHEMICAL SOCIETY 200409 IN, vol. 81, no. 9, September 2004 (2004-09), pages 778-780, XP009160035, ISSN: 0019-4522
- CHRISTOS M. DOULGKERIS ET AL: "Compounds against inflammation and oxidative insult as potential agents for neurodegenerative disorders", MEDICINAL CHEMISTRY RESEARCH, 1 January 2011 (2011-01-01), XP55029379, ISSN: 1054-2523, DOI: 10.1007/s00044-011-9726-x
- GALANAKIS DIMITRIOS ET AL: "Synthesis and pharmacological evaluation of amide conjugates of NSAIDs with L-cysteine ethyl ester, combining potent antiinflammatory and antioxidant properties with significantly reduced gastrointestinal toxicity", BIOORGANIC & MEDICINAL CHEMISTRY LETTERS, PERGAMON, ELSEVIER SCIENCE, GB, vol. 14, no. 14, 16 July 2004 (2004-07-16) , pages 3639-3643, XP002366913, ISSN: 0960-894X, DOI: 10.1016/J.BMCL.2004.05.025
- HORST WEBER ET AL: PHARMACEUTICAL RESEARCH, vol. 18, no. 5, 1 January 2001 (2001-01-01), pages 600-607, XP55029405, ISSN: 0724-8741, DOI: 10.1023/A:1011021024254
- PIGNATELLO R ET AL: "Synthesis and In Vitro skin permeation of naproxen conjugates with [alpha] -alkylamino acids", CURRENT DRUG DELIVERY, BENTHAM SCIENCE PUBLISHERS, HILVERSUM, NL, vol. 2, no. 2, 1 April 2005 (2005-04-01), pages 185-189, XP009160034, ISSN: 1567-2018
- LEVIT G L ET AL: "Synthesis and antiinflammatory and analgesic activity of naproxen amides with amino acid derivatives", PHARMACEUTICAL CHEMISTRY JOURNAL 20020501 US LNKD- DOI:10.1023/A:1020561127636, vol. 36, no. 5, 1 May 2002 (2002-05-01), pages 232-236, XP009160063, ISSN: 0091-150X
- WEISCHER C H: "Some histochemical aspects of the carrageenin induced inflammation", ARCHIV FUR EXPERIMENTELLE VETERINARMEDIZIN 1975 DD, vol. 29, no. 5, 1975, pages 769-776, XP009185711, ISSN: 0003-9055

## Description

### FIELD OF THE INVENTION

The present invention refers to amides of NSAIDs, preferably of naproxen amino acids esters having the chemical structure of formula I 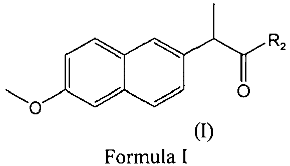 wherein R₂ is an amino acid ester residue, or any racemic form, prodrug, enantiomer, analog of the substance, or pharmaceutical composition comprising such compound for use as a medicament to treat or prevent cancer. The compounds of the inventions may be used for the treatment or prevention of a cancer in mammals. Preferably, the compounds may be used for the treatment or prevention of colorectal cancers. Also disclosed are methods for the treatment of a proliferative disease or disorder, comprising administering to a mammal in need thereof an effective amount of a therapeutic agent, wherein the therapeutic agent comprises such amide of naproxen amino acids esters.

### BACKGROUND OF THE INVENTION

Cancer is one of the most dreaded diseases of mankind. It is a leading cause of death throughout the world. Currently, one in four deaths in the United States is due to cancer.¹ More than ten million new cancer cases occur annually; roughly half of which are in the developed countries. Malignant disease causes over six million deaths a year. ^{2,3}

The pattern of cancer in the Kingdom of Saudi Arabia has been investigated in which a total of 7251 new cases of cancer (4117 males and 3134 females) were seen over a period up to six years at King Faisal Specialist Hospital (Riyadh). Non-Hodgkins lymphoma, lung, liver, stomach, nasopharynx and prostate cancers are the most common among males, while breast (18% of all cancers in Saudi women), cervix and ovary cancers are the most common among females. While locally advanced breast cancer disease is unusual in western countries, it constitutes more than 40% of all non-metastatic breast cancer in the Kingdom of Saudi Arabia. The incidence of colorectal cancer (CRC) is second to breast cancer. Cervical carcinoma ranks as the eighth most common cancer, accounting for 3.6% of all cancers in Saudi females.⁴⁻⁷

Thus, CRC is a major cause of mortality and morbidity worldwide. In the Kingdom of Saudi Arabia, the incidence of CRC is increasing whereas the incidence rate was originally lower than in Western countries. According to the latest statistics, CRC is considered the second most common cancer among Saudi males and the third most common among Saudi females.^{8,9}

Epidemiologic, clinical, and experimental studies have shown that certain nonsteroidal anti-inflammatory drugs (NSAID) and cyclooxygenase (COX)-2 selective inhibitors, display strong evidence of cancer chemopreventive efficacy. Population based studies, for example, have shown that aspirin and to a greater extent, non aspirin NSAIDs can significantly reduce the incidence and risk of death from CRC.^{10,11} NSAIDs are also effective in reducing existing polyps in familial adenomatous polyposis patients. Consistent with these clinical observations, NSAIDs also inhibit tumorigenesis in various animal models of colorectal cancer. A similar inverse association has been described for other cancers including cancer of the breast, ovary, rectum, and esophagus.^{12,13} For prostate cancer, there are a few studies showing a weak (but not statistically significant) inverse association between NSAID use and cancer.¹⁴ However, a recent report by Nelson and Harris,¹⁵ indicated a 70% reduction in the risk of prostate cancer among NSAID users. A community population-based study,¹⁴ showed that there is a significant reduction in prostate cancer risk among men aged 60 years or older who were daily users of NSAIDs. Interestingly, the study found the strongest inverse association with an 83% reduction in risk among men of age 70 years or older. The authors attributed this to the age group with the highest use of NSAIDs and suggested NSAIDs as potential chemopreventive agents for prostate cancer. However, the molecular mechanism(s) responsible for their antineoplastic activity remains to be elucidated.

Recently two randomised placebo controlled trials showed that aspirin reduced the risk of colorectal adenomas in populations with an intermediate risk of developing carcinoma.^{16,17}.

However, before deciding whether aspirin should be recommended for chemoprevention in persons with a history of colorectal cancer, the clinical importance of these two studies requires further consideration given the potentially fatal toxicities associated with long-term use of NSAIDs.

Despite the intensive research that has been aimed at the investigation of NSAIDs as cancer chemopreventive agents, their clinical usefulness is still restricted by their gastrointestinal side effects, including gastric irritation, ulceration, bleeding, and perforation and in some cases may develop into life threatening conditions.¹⁸ Previous studies have revealed that many efforts had been made to synthesize amino acid ester, glycolamide ester, and amide derivatives of NSAIDs using various amines but few attempts were made to develop amide derivatives using amino acids.¹⁹ However, only inflammatory effects were shown with amide derivatives using amino acids.¹⁹

Most NSAIDs act as non-selective inhibitors of the enzyme, cyclooxygenase (COX), inhibiting both cyclooxygenase-1 (COX-1) and cyclooxygenase-2 (COX-2) isozymes.

COX-1 is a constitutively expressed enzyme with a "house-keeping" role in regulating many normal physiological processes. One of these is in the stomach lining, where prostaglandins serve a protective role to prevent damage from gastric acid. Prostaglandins also play an important role in kidney function whereby COX-1 inhibition can result in kidney failure.

When non-selective COX-1/COX-2 inhibitors (such as aspirin, ibuprofen, and naproxen) lower stomach prostaglandin levels, their protective effects are lost, whereby ulcers of the stomach or duodenum can form that result in potentially fatal internal bleeding, ulcers, or perforations. COX-2 is an inducible enzyme that is selectively expressed in inflammation, and selective inhibition by COX-2 inhibitors (e.g. celecoxib) can result in less gastrointestinal and kidney toxicity compared with conventional NSAIDs. However, recent studies have shown that COX-2 inhibitors are associated with increased risk of stroke and myocardial infarction.

So far, no effective non-selective amides of NSAID amino acids esters derivatives could be found and the anticancer efficacy of these substances remain unknown.

US 6,207,700 discloses a method for cancer treatment in a warm blooded vertebrate animal, comprising administering a carboxylic acid secondary amide derivative of a compound, which could be a NSAID. Said compounds do not inhibit either COX-1 or COX-2 and were designed by substituting the carboxylic acid moiety with an amide linker to add a bulkier group, which prevented COX-1 and COX-2 binding, but retained anticancer activity, and are described as being useful for the prevention or treatment of cancer.

US 2007/0292352 discloses a cyclooxygenase-2-selective therapeutic and/or diagnostic agent comprising an active agent and a derivative of a NSAID useful for the treatment of cancer.

One object of the invention is to provide compounds, or drugs that are useful in treating cancer, and which have reduced side effects.

### SUMMARY OF THE INVENTION

The object of the present invention is solved by the subject-matter as defined in the claims of this application.

The object of the invention is solved by a compound having the chemical structure of any one of formula III to formula XIV: 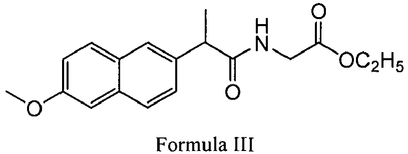 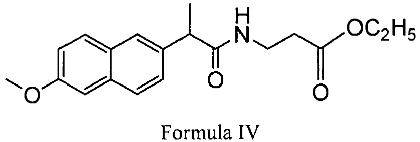 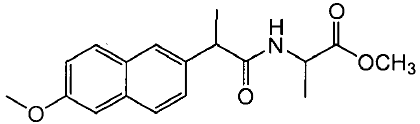 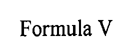 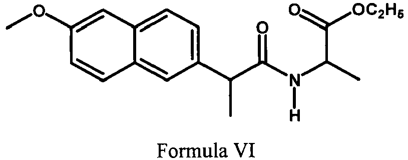 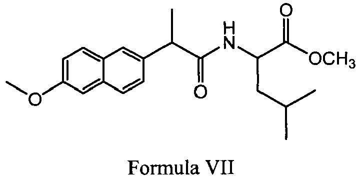 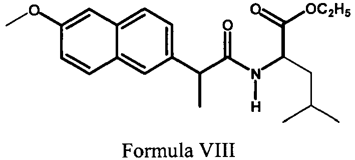 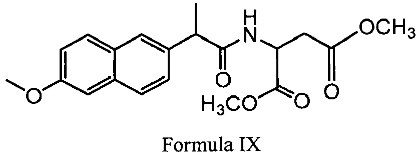 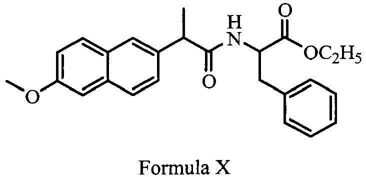 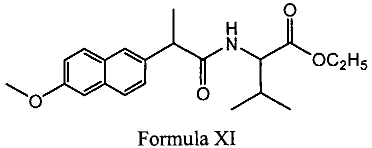 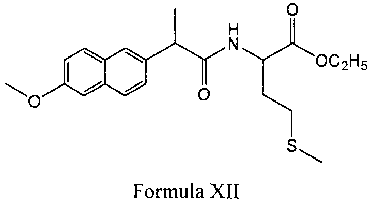 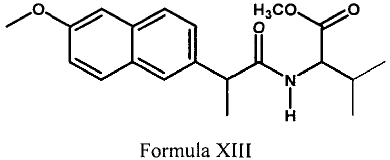 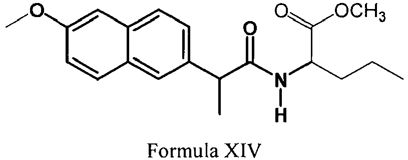 or any racemic form, or enantiomer of the substance for use in the prevention or treatment of cancer.

In a preferred embodiment, the compound has the chemical structure of any one of formula III to V, VII, or IX to XII: 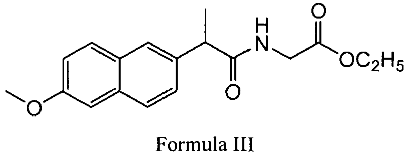 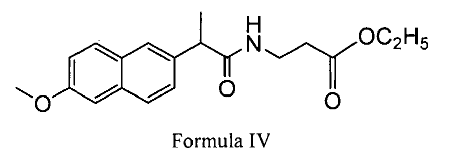 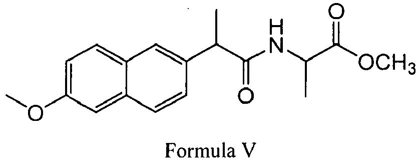 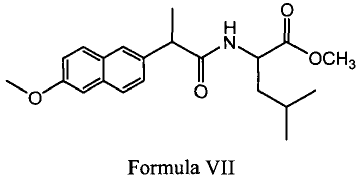 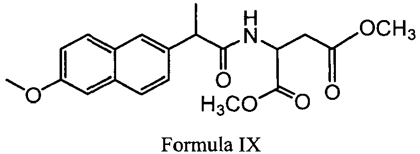 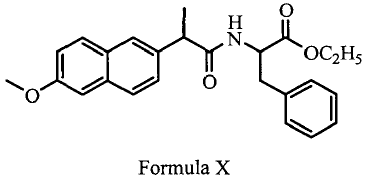 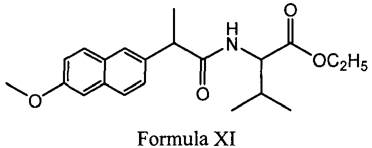 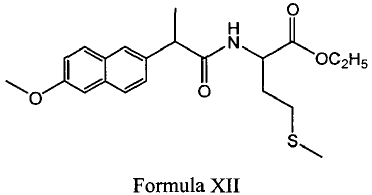 or any racemic form, or enantiomer of the substance for use in the prevention or treatment of cancer. In a preferred embodiment, the compound has the chemical structure of any one of formula III, VII, X, XI, or XII.

The object of the invention is further solved by a compound as described herein for use in the prevention or treatment of cancer. Preferably, the cancer is colorectal cancer, prostate cancer, lung cancer, breast cancer, brain cancer, cervical cancer, Hodgkin's lymphoma, kidney cancer, leukemia, liver cancer, non-Hodgkin's lymphoma, ovarian cancer, skin cancer, testicular cancer, thyroid cancer, uterine cancer, esophageal, or bladder cancer, wherein preferably the cancer is colorectal cancer.

The object of the invention is also solved by a pharmaceutical composition comprising a compound as described herein and a pharmaceutical acceptable carrier for use as a medicament for the treatment of cancer. The pharmaceutical composition as described herein is for use as a medicament for the treatment of cancer, preferably for the treatment of prostate cancer, lung cancer, colorectal cancer, breast cancer, brain cancer, cervical cancer, Hodgkin's lymphoma, kidney cancer, leukemia, liver cancer, non-Hodgkin's lymphoma, ovarian cancer, skin cancer, testicular cancer, thyroid cancer, uterine cancer, or bladder cancer, wherein more preferably the cancer is colorectal cancer.

The dosage form of the pharmaceutical composition is a tablet, lozenge, pill, dragee, capsule, liquid, gel, syrup, slurry, suspension, solution or emulsion. The pharmaceutical composition as described herein or the compound as described herein may be for oral, rectal, transmucosal, transdermal, intestinal, parenteral, intramuscular, intrathecal, direct intraventricular, intravenous, intraperitoneal, intranasal, intraocular, or subcutaneous administration.

Also disclosed is a method for the treatment of a proliferative disease or disorder, comprising administering to a subject in need thereof an effective amount of a therapeutic agent, wherein the therapeutic agent comprises a compound as described herein, or a pharmaceutical composition as described herein. Preferably, the proliferative disease or disorder is prostate cancer, lung cancer, colorectal cancer, breast cancer, brain cancer, cervical cancer, Hodgkin's lymphoma, kidney cancer, leukemia, liver cancer, non-Hodgkin's lymphoma, ovarian cancer, skin cancer, testicular cancer, thyroid cancer, uterine cancer, or bladder cancer, preferably wherein proliferative disease is colorectal cancer.

The compounds pharmaceutical compositions, and methods as described herein are be useful for a subject in need thereof, whereby a subject in need thereof is an animal, or human being, preferably a mammal, more preferably a human being.

### DETAILED DESCRIPTION OF THE INVENTION

It is to be understood that this invention is limited only by the appended claims. All technical and scientific terms used herein have the same meanings as commonly understood by one of ordinary skill in the art, unless described otherwise.

An "ester" is a chemical compound derived by reacting an oxoacid with a hydroxyl compound such as an alcohol or phenol. Esters are most commonly from carboxylic acids and alcohols. As disclosed herein, any hydroxyl compound, alcohol, phenol, or derivatives thereof may be useful for the present invention.

An "alkyl" or "alkyl group" is a functional group or side-chain that, like an alkane, consists solely of single-bonded carbon and hydrogen atoms. Alkyls as disclosed herein are methyl, ethyl, propyl, butyl, pentyl, hexyl, heptyl, octyl, nonyl, decanyl, undecanyl, dodecanyl, icosanyl, triacontanyl, tetracontanyl, or pentacontanyl groups, or any other alkyl known to the skilled person. An "aralkyl" is any univalent radical derived from an alkyl radical by replacing one or more hydrogen atoms by aryl groups.

An "amino acid" in the context of this invention can be any natural, synthetic, or artificial amino acid, and their respective optical isomers, analogs, or modifications. Natural amino acids comprise alanine, valine, leucine, isoleucine, proline, tryptophane, phenylalanine, methionine, glycine, serine, tyrosine, threonine, cysteine, asparagine, glutamine, aspartatic acid, glutamic acid, lysine, arginine, and histidine, or any other amino acid commonly understood by one of ordinary skill in the art.

A "nonsteroidal anti-inflammatory drug" ("NSAID") can be fenamic acids, such as flufenamic acid, niflumic acid, and mefenamic acid; indoles, such as indomethacin, sulindac, and tolmetin; phenylalkanoic acids, such as suprofen, ketorolac, flurbiprofen, and ibuprofen; and phenylacetic acids, such as diclofenac. Also, the NSAID can be aceloferac, etodolic acid, loxoprofen, alcofenac, fenbufen, meclofenamate, amfenac, fenclofenac, naproxen, benoxaprofen, fenclorac, orpanoxin, bromfenac, fenoprofen, pirprofen, carprofen, fleclozic acid, pranoprofen, clidanac, indoprofen, tolfenamic acid, diflunisal, isofezolac, zaltoprofen, efenamic acid, ketoprofen, zomopirac, or any other NSAID commonly understood by one of ordinary skill in the art. Preferably, the NSAID is naproxen.

The principle advantage of the invention is to provide non-COX inhibitory derivatives of NSAIDs that have increased potency and selectivity to inhibit tumor cell growth compared with the parent compound and therefore have the potential for improved efficacy and reduced toxicity.

Accordingly, the current invention describes design, synthesis and colon tumor cell inhibitory activity of compounds that are amide prodrugs of a nonsteroidal anti-inflammatory drug (NSAID) linked to an amino acid ester residue via an amide bond, preferably wherein the amino acid residue is glycine ethyl ester, alanine methyl ester, alanine ethyl ester, leucine methyl ester, leucine ethyl ester, aspartic acid dimethyl ester, phenylalanine ethyl ester, valine ethyl ester, methionine ethyl ester, valine methyl ester, or methyl amino butyrate, preferably wherein the amino acid ester residue has an alkyl or aralkyl residue, which is preferably methyl or ethyl, for use as a medicament for the treatment of a proliferative disease or disorder. Alkyl or aralkyl residues both increase the lipophilicity and reduce the irritability of gastro-intestinal tract of the molecule compared with the free acid.

Also disclosed are compounds which have the following general structure of formula (I): 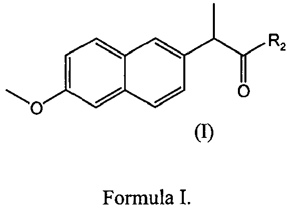 wherein R₂ is an amino acid ester residue.

The salient features of the usefulness of conjugation of amino acids are as follows:²⁰
(i) Amino acids are normal dietary constituent and they are non-toxic in moderate doses as compared to other chemical substitutions;
(ii) Amino acids have healing effect on gastric lesions produced by NSAIDs;
(iii) A drug with a free carboxyl group can be derivatized into corresponding esters or amide of amino acids, so as to alter the physical properties of a parent drug with one or more of the hydrolase enzymes serving as the *in vivo* reconversion site(s);
(iv) Being a nutritional substance, the use of amino acids as a derivatizing group might also permit more specific targeting site for enzymes involved in the terminal phase of digestion;
(v) By using different types of amino acids like non-polar, polar, acidic and basic, the drug molecule can be made more or less polar, or more or less soluble in a given solvent.

Accordingly, the disclosed substances may also have various amino acid ester residues, like, without limiting, glycine ethyl ester, alanine methyl ester, alanine ethyl ester, leucine methyl ester, leucine ethyl ester, aspartic acid dimethyl ester, phenylalanine ethyl ester, valine ethyl ester, methionine ethyl ester, valine methyl ester, or methyl amino butyrate. This allows an individual medication by at the same time having medicaments with reduced side effects.

These substances show an effect in inhibiting cancer cell growth. In particular, these substances inhibit the growth of colon adenocarcinoma cells (Table 2). However, the anti-cancer effect of these substances is suitable for the prevention and treatment for prostate cancer, lung cancer, colorectal cancer, breast cancer, brain cancer, cervical cancer, Hodgkin's lymphoma, kidney cancer, leukemia, liver cancer, non-Hodgkin's lymphoma, ovarian cancer, skin cancer, testicular cancer, thyroid cancer, uterine cancer, or bladder cancer, or any proliferative disease or disorder as well.

The compounds disclosed herein do not inhibit either COX-1 or COX-2 and therefore are likely to have less side effects than NSAIDs and COX-2 inhibitors.

Accordingly, the compounds disclosed herein will minimize these side effects and thus can be valuable candidates for the prevention or treatment of cancer. The gastrointestinal mucosal injury problems have been overcome by a derivatization of carboxylic function of NSAIDs into amides. The substances have reduced side effects and thus help to protect the stomach mucosa. Accordingly, the substances disclosed herein are also useful for an individual medication and therapy.

The compounds of the invention that are amides of naproxen having an amino acid ester residue with an alkyl or aralkyl residue, preferably methyl or ethyl, have particular effectiveness in the treatment and prevention of proliferative diseases. These compounds are substances with one of the following structures of formula III to XIV of Table 1:

**Table 1**

| **Compound/ Formula** | **Structure** |
|---|---|
| III | 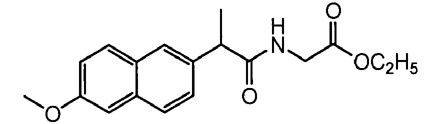 |
| IV | 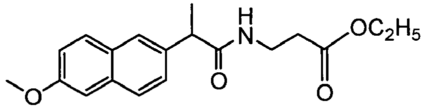 |
| V | 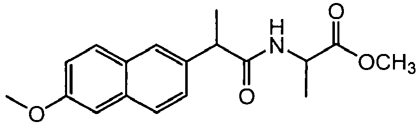 |
| VI | 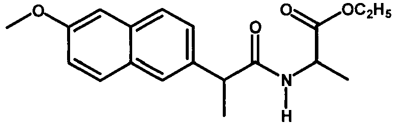 |
| VII | 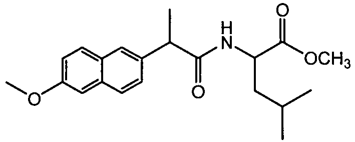 |
| VIII | 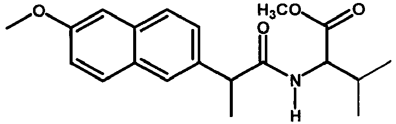 |
| IX | 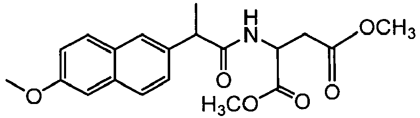 |
| X | 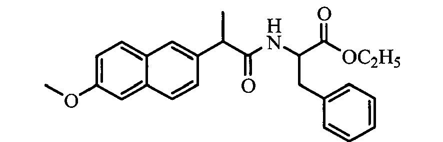 |
| XI | 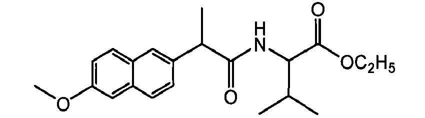 |
| XII | 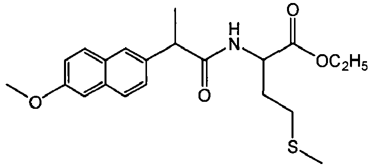 |
| XIII | 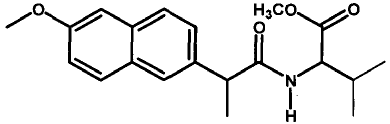 |
| XIV | 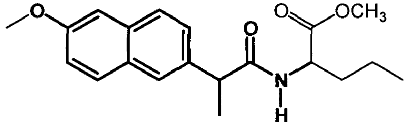 |

Table 1: Selected amides of naproxen amino acid esters of the present invention.

The compounds of Table 1 show efficient carcinoma cell growth inhibition (also see Example 2 and Table 3).

The compounds of Table 1 have the same dietary and therapeutic advantages of amides of an NSAID amino acid esters, in particular of amides of naproxen amino acids esters, as described herein. Also, any racemic form, enantiomer, prodrug, or analog of these compounds are disclosed herein.

The compounds as disclosed herein are effective in inhibition of carcinoma cell growth. This effect is shown in the human HT-29 colon adenocarcinoma cell line (also see Example 2). Accordingly, the compounds described herein, especially the compounds of Table 2, and 3, are for use in the treatment of cancer in particular for use in the treatment of prostate cancer, lung cancer, colorectal cancer, breast cancer, brain cancer, cervical cancer, Hodgkin's lymphoma, kidney cancer, leukemia, liver cancer, non-Hodgkin's lymphoma, ovarian cancer, skin cancer, testicular cancer, thyroid cancer, uterine cancer, or bladder cancer. In a most preferred embodiment of the invention, the cancer is colorectal cancer.

**Table 2**

| **Compound/ Formula** | **Structure** |
|---|---|
| III | 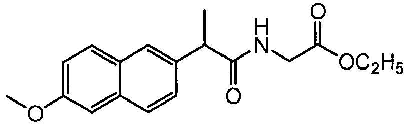 |
| IV | 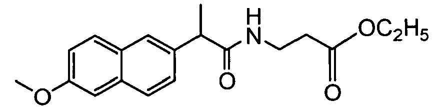 |
| v | 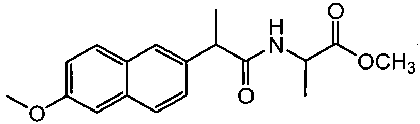 |
| VI | 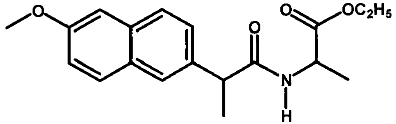 |
| VII | 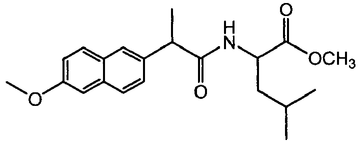 |
| IX | 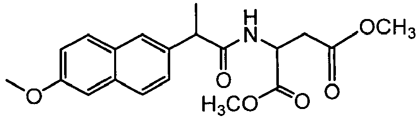 |
| X | 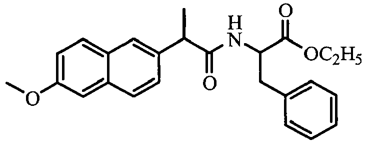 |
| XI | 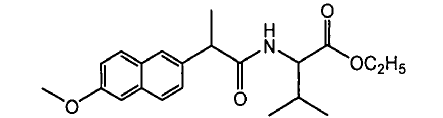 |
| XII | 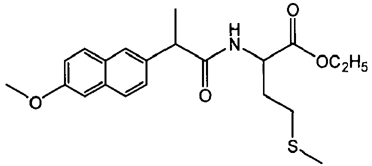 |

Table 2: Selected amides of naproxen amino acid ester prodrugs of the present invention.

Maximum activity was shown for compound III, with the amide of glycine ethyl ester; compound XI, with the amide of leucine ethyl ester; compound X, with the amide of phenylalanine ethyl ester; compound VII, with the amide of leucine methyl ester and compound XII, with the amide of methionine methyl ester. These data (cf. Example 2; Table 3) show that these substances are in particular effective in the prevention or treatment of a proliferative disease, and in particular for the prevention or treatment of colorectal cancer.

While it is possible that, for use in therapy, a compound according to the invention may be administered as the raw chemical, it is preferable to present the active ingredient as a pharmaceutical composition. The carrier and/or excipient of the pharmaceutical composition must be "acceptable" in the sense of being compatible with the other ingredients of the formulation and not deleterious to the recipient thereof.

The present invention also relates to a pharmaceutical composition comprising a compound of and a pharmaceutical acceptable carrier for use as a medicament for the treatment of cancer, preferably for the treatment of prostate cancer, lung cancer, colorectal cancer, breast cancer, brain cancer, cervical cancer, Hodgkin's lymphoma, kidney cancer, leukemia, liver cancer, non-Hodgkin's lymphoma, ovarian cancer, skin cancer, testicular cancer, thyroid cancer, uterine cancer, or bladder cancer, wherein more preferably the cancer is colorectal cancer.

Due to the advantages of the compounds as described herein, especially due to the healing effects of the amino acid residue, the dietary compatibility, the possibility of specific targeting to enzymes, and the flexibility of designing compounds with different polarity and other chemical characteristics, such pharmaceutical composition may be designed for individual medication or treatment and show high compatibility.

Dosage forms of the pharmaceutical compositions as disclosed herein can be tablets, lozenges, pills, dragees, capsules, liquids, gels, syrups, slurries, suspensions, solutions or emulsions. Accordingly, the compounds and/or the pharmaceutical compositions can be administered orally, rectally, transmucosally, transdermally, intestinally, parenterally, intramuscularly, intrathecally, direct intraventricularly, intravenously, intraperitoneally, intranasally, intraocularly, or subcutaneously.

The advantages of the compounds and pharmaceutical composition as disclosed herein also apply to a method for the treatment of a proliferative disease or disorder, comprising administering to a subject in need thereof an effective amount of a therapeutic agent, wherein the therapeutic agent comprises a compound and/or pharmaceutical composition as described herein. Accordingly, these methods can be adapted to the individual need of a subject in need thereof.

The compounds, pharmaceutical compositions, and methods as described herein may be useful for a subject in need thereof, whereby a subject in need thereof is an animal, or human being, preferably a mammal, more preferably a human being.

The compounds, and pharmaceutical compositions, as disclosed herein can be used for the treatment of a proliferative disease or disorder, which preferably is prostate cancer, lung cancer, colorectal cancer, breast cancer, brain cancer, cervical cancer, Hodgkin's lymphoma, kidney cancer, leukemia, liver cancer, non-Hodgkin's lymphoma, ovarian cancer, skin cancer, testicular cancer, thyroid cancer, uterine cancer, or bladder cancer, more preferably colorectal cancer. Thus, the methods provided are a better and effective tool to curtail the damages of cancer, in particular colorectal cancer, and contribute to an improved and more individual treatment and healing of such disease. Also, having reduced side effects, the compounds, pharmaceutical compositions, and methods as disclosed herein, have an increased physiological compatibility.

The invention shall be described in more detail on the basis of the following examples and with reference to the attached Figures, without being limited thereto.

### DESCRIPTION OF THE FIGURES

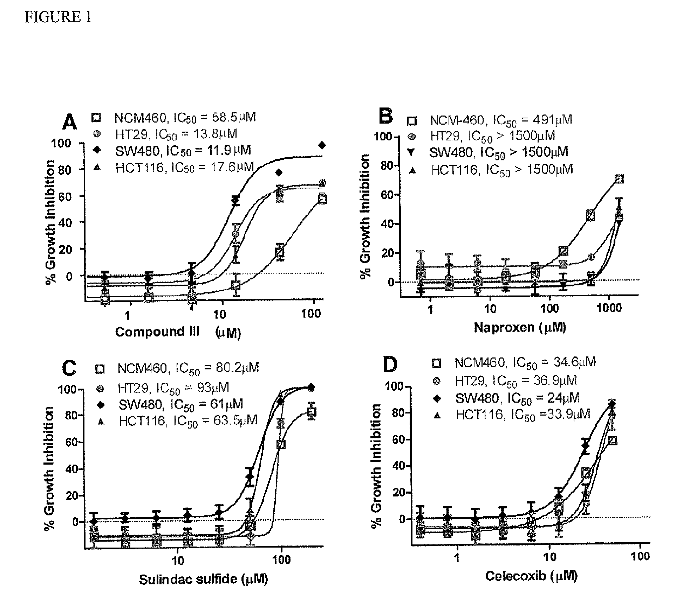

Figure 1: Tumor cell growth inhibitory activity and selectivity of (A) compound III, (B) naproxen (non-selective COX-1 and COX-2 inhibitor), (C) sulindac sulfide (non-selective COX-1 and COX-2 inhibitor) and (D) celecoxib (selective COX-2 inhibitor). All compounds were evaluated for tumor cell growth inhibitory activity in the three human colon tumor cell lines, HT29, SW480, and HCT118 and for tumor selectivity using normal human colonocytes (NCM460). Note the improved potency (>1000 fold) of compound III compared with naproxen, along with improved tumor selectivity. Compound III was also appreciably more potent and tumor selective compared with the known cancer chemopreventive NSAIDs, sulindac sulfide and celecoxib.

### EXAMPLES

### EXAMPLE 1

### Materials and Methods

### 1.1. Synthesis

The general procedures for the preparation of the target amides of NSAIDs with amino acid esters is described in scheme 1:

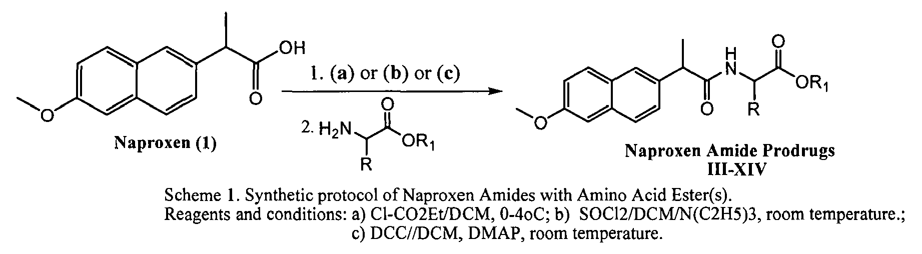

The target compounds, could be synthesized via the reaction of the activated carboxylic acid moiety of the appropriate NSAID with amino acid ester. Activation of NSAID carboxylic acid function group can be achieved by forming the mixed anhydride using ethyl chloroformate/triethylamine in dichloromethane (DCM) at 0-4°C. Target compounds can also be obtained by coupling the NSAID with the amino acid ester in the presence of dicyclohexyldicarbdiimide (DCC) as the carboxylate activator and 4-dimethylaminopyridine (DMAP) as catalyst as illustrated by scheme 1. Activation of the carboxylic acid group of NSAID can also be achieved by forming the corresponding acid chloride with the thionyl chloride in DCM followed by its reaction with an amino acid ester in the presence of one equivalent of triethylamine.

The synthesized compounds were purified by flash chromatography and crystallized from ethanol. The structures were confirmed by spectroscopic methods of analyses.

### 1.2. In vitro tumor cell growth inhibitory activity of naproxen derivatives

### 1.2.1. Cells and Cell Culture:

The human colon adenocarcinoma cell lines were obtained from ATCC and grown under standard cell culture conditions in RPMI 1640 medium containing 5% fetal bovine serum (FBS) at 37°C in a humidified atmosphere with 5% CO₂.

### 1.2.2. Growth Assay:

Tissue culture treated 96-well microtiter plates were seeded at a density of 5,000 cells/well. For growth inhibition assays, cells were incubated at 37°C for 18-24 hours, treated with the specified compound or vehicle (0.1% DMSO final) control, and incubated at 37°C an additional 72 hours. The effect of treatment on cell viability was determined using the luminescent Cell Titer Glo Assay (Promega).

### EXAMPLE 2

HT-29 human colon adenocarcinoma cells were treated with compounds of the invention and grown under standard cell culture conditions. Measurement of viable cell number revealed promising tumor cell growth inhibitory activity (Table 3).

**Table 3**

| **Compound/ Formula** | **Structure** | **HT-29 Growth Inhibition IC₅₀ (µM)** |
|---|---|---|
| III | 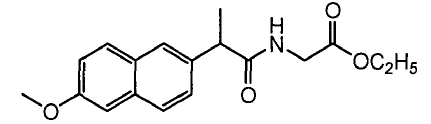 | 14.6 |
| IV | 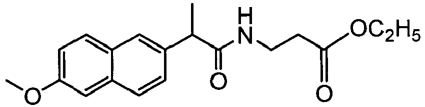 | 123.4 |
| VII | 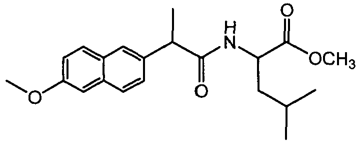 | 34.5 |
| IX | 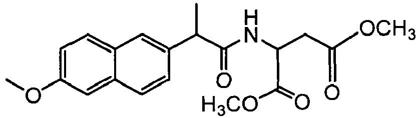 | 120.7 |
| X | 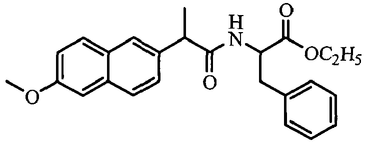 | 33.6 |
| XI | 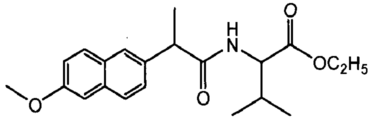 | 29.4 |
| XII | 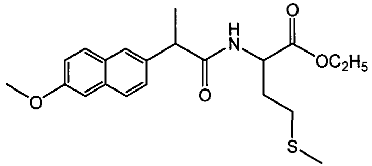 | 49.3 |

Table 3: Half maximal inhibitory concentration (IC₅₀)

Maximum activity was shown for compound III, with the amide of glycine ethyl ester (IC₅₀= 14.6 µM); compound XI, with the amide of leucine ethyl ester, (IC₅₀= 29.4 µM); compound X, with the amide of phenylalanine ethyl ester (IC₅₀= 33.6 µM); compound VII, with the amide of leucine methyl ester (IC₅₀= 34.5 µM) and compound XII, with the amide of methionine methyl ester (IC₅₀= 49.3 µM).

Accordingly the synthesized compounds derivatives of Naproxen III to XII are potential candidates for both treatment and chemoprevention of colorectal cancer.

### EXAMPLE 3

All compounds were evaluated for tumor cell growth inhibitory activity in the three human colon tumor cell lines, HT29, SW480, and HCT118 and for tumor selectivity using normal human colonocytes (NCM460). Note the improved potency (>1000 fold) of compound III compared with naproxen, along with improved tumor selectivity. Compound III was also appreciably more potent and tumor selectivity compared with the known cancer chemopreventive NSAIDs, sulindac sulfide and celecoxib (see Figure 1).

### REFERENCES

1. Ahmedin, J.; Siegel, R.; Ward, E.; Hao, Y.; Xu, J.; Murray, T.; Thun, M. J. CA Cancer J Clin 2008, 58:71.
2. Sinha, R.; El-Bayoumy, K. Current Cancer Drug Targets 2004, 4: 13.
3. Cozzi, P.; Mongelli, N.; Suarato, A. Curr. Med. Chem. - Anti-Cancer Agents 2004, 4:93.
4. L. S. Abdulah; Ann. Saudi. Med. 2007, 27:268-272.
5. M. Ezzeldin, M. Ibrahim, A. Ahmed, A. Zeeneldin, R. Tawfik, A.M. El-Khodary, A.M. Al-Gahmi, M.B. Bin Sadiq; Saudi J Gastroenterol. 2008, 14: 178-182.
6. D.M. Parkin, F. Bray, J. Ferlay, P. Pisani; Global cancer statistics, (2002). CA Cancer J Clin. 2005, 55:74-108.
7. E. Ibrahim, S.B. Bin, L. Banjar, S. Awadalla, M.S. Abomelha; Hematol Oncol Stem Cell Ther. 2008, 1:210-5.
8. S. Uddin, P. Bavi, A. R. Hussain, G. Alsbeih, N. Al-Sanea, A. AbdulJabbar, L. H. Ashari, S. Alhomoud, F. Al-Dayel, M. Ahmed, K. S. Al-Kuraya, Leptin Receptor Expression in Middle Eastern Colorectal Cancer and it's Potential Clinical Implication. http://carcin.oxfordjournals.org. downloaded on November 22 (2010).
9. P.P. Bavi, J.A. Abubaker, Z.D. Jehan, N.A. Al-Jomah, A.K. Siraj, S.R. Al-Harbi, V.L. Atizado, A.S. Abduljabbar, S.J. Alhomoud, L.H. Ashari, F.H. Al-Dayel, S. Uddin, K.S. Al-Kuraya and N.A. Alsanea,. Saudi Med J, 2008, 29:75-80.
10. M.J. Thun, S.J. Henley, C. Patrono, J Natl Cancer Inst 2002, 94:252-6.
11. T.A. Chan, Lancet Oncol, 2002, 3:166-74.
12. G. N. Levy, H. Prostaglandin, FASEB J., 1997, 11: 234-247.
13. S.S. Husain, I. L., Szabo, A. S. Tarnawski, Am. J. Gastroenterol., 2002, 97: 542-553.
14. R. O. Roberts, D.J. Jacobson, C. J. Girman, T. Rhodes, M.M. Lieber, S. J. A. Jacobsen, Mayo Clin. Proc., 2002, 77: 219-225.
15. J.E.Nelson, R. E., Harris, Oncol. Rep., 2000, 7: 169-170.
16. R.S. Sandler, S. Halabi, J.A. Baron et al. N Engl J Med, 2003, 348:883-890.
17. J.A. Baron, B.F. Cole, R.S. Sandler et al. NEngl JMed, 2003, 348:891-899.
18. T. S. Robert, J. V. Ronald. Mechanisms of non-steroidal anti-inflammatory drug-induced gastric damage. Am. J. Med., 1989, 86:449-458.
19. A. Mishra, R. Veerasamy, P. K. Jain, V. K. Dixit and R. K. Agrawal, J. Braz. Chem. Soc., 2008,19: 89-100.
20. M. Rooseboom, J.N. Commandeur, N. P. Vermeulen, Pharmacol. Rev., 2004, 56:53-102.

## Claims

1. A compound having the chemical structure of any one of formula III to formula XIV: 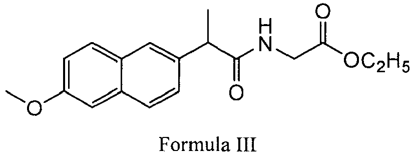 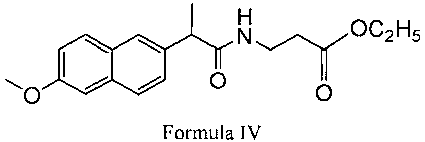 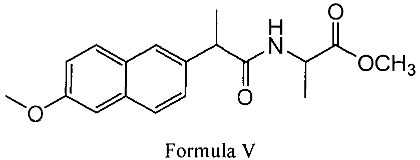 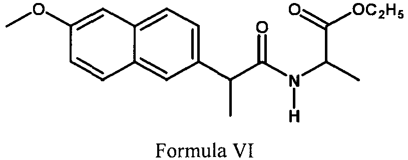 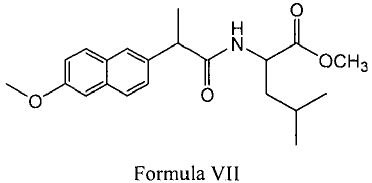 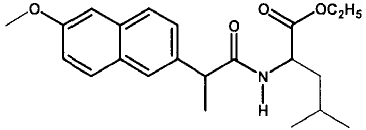 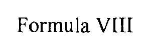 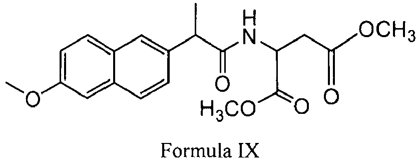 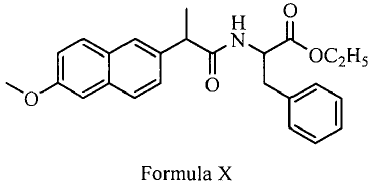 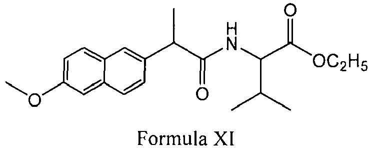 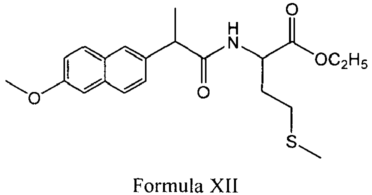 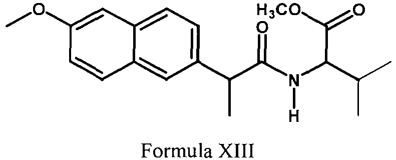 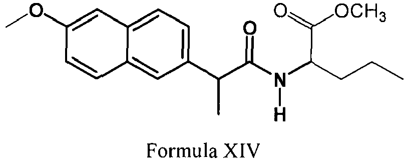 or any racemic form or enantiomer of the substance, **for use in the prevention or treatment of cancer.**

2. The compound for use according to claim 1, having the chemical structure of any one of formula III to V, VII, or IX to XII: 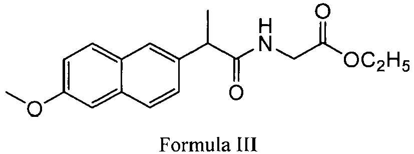 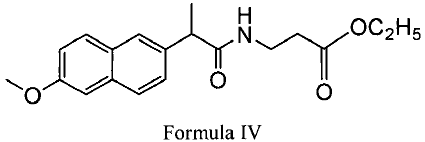 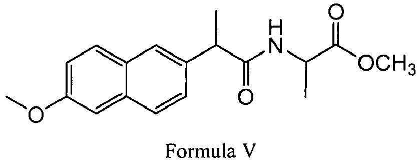 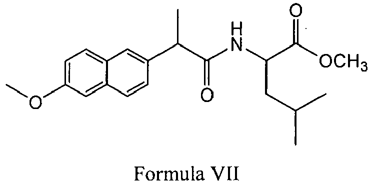 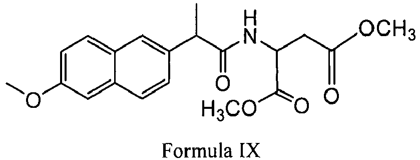 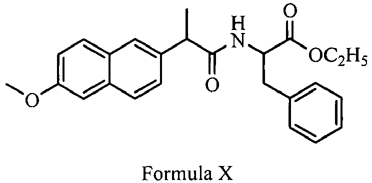 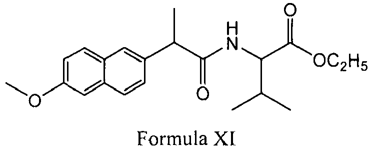 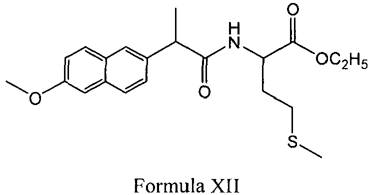 or any racemic form or enantiomer of the substance.

3. The compound for use according to claim 1, having the chemical structure of any one of formula III, VII, X, XI, or XII.

4. The compound for use according to any one of claim 1 to 3, wherein the cancer is prostate cancer, lung cancer, colorectal cancer, breast cancer, brain cancer, cervical cancer, Hodgkin's lymphoma, kidney cancer, leukemia, liver cancer, non-Hodgkin's lymphoma, ovarian cancer, skin cancer, testicular cancer, thyroid cancer, uterine cancer, or bladder cancer, wherein preferably the cancer is colorectal cancer.

5. A pharmaceutical composition comprising a compound for use according to any one of claims 1 to 4 and a pharmaceutical acceptable carrier for use as a medicament for the treatment of cancer.

6. The pharmaceutical composition of claim 5 for use as a medicament for the treatment of cancer, wherein the cancer is preferably prostate cancer, lung cancer, colorectal cancer, breast cancer, brain cancer, cervical cancer, Hodgkin's lymphoma, kidney cancer, leukemia, liver cancer, non-Hodgkin's lymphoma, ovarian cancer, skin cancer, testicular cancer, thyroid cancer, uterine cancer, or bladder cancer, wherein more preferably the cancer is colorectal cancer.

7. The pharmaceutical composition **for use according** to any one of claims **5 to 6,** wherein the pharmaceutical composition is to be administered in a dosage form and the dosage form is a tablet, lozenge, pill, dragee, capsule, liquid, gel, syrup, slurry, suspension, solution or emulsion.

8. The pharmaceutical composition **for use according to** any one of claim **5 to 7** for oral, rectal, transmucosal, transdermal, intestinal, parenteral, intramuscular, intrathecal, direct intraventricular, intravenous, intraperitoneal, intranasal, intraocular, or subcutaneous administration.

9. A therapeutic agent for use in a method for the treatment of cancer, comprising administering to a subject in need thereof an effective amount of said therapeutic agent, wherein the therapeutic agent comprises a compound according to any one of claims 1 to 4, or a pharmaceutical composition according to any one of claims **5 to 8.**

10. The therapeutic agent for use according to claim **9,** wherein the therapeutic agent comprises a compound according to claim 1 to **4,** and wherein the cancer is prostate cancer, lung cancer, colorectal cancer, breast cancer, brain cancer, cervical cancer, Hodgkin's lymphoma, kidney cancer, leukemia, liver cancer, non-Hodgkin's lymphoma, ovarian cancer, skin cancer, testicular cancer, thyroid cancer, uterine cancer, or bladder cancer, wherein preferably said cancer is colorectal cancer.

## Patentansprüche

1. Verbindung mit der chemischen Struktur einer der Formeln III bis XIV: 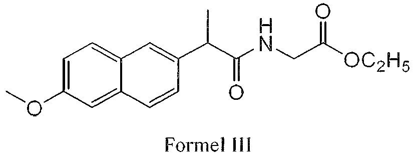 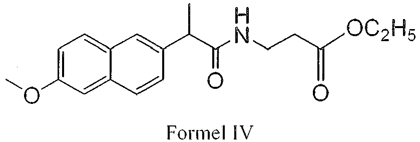 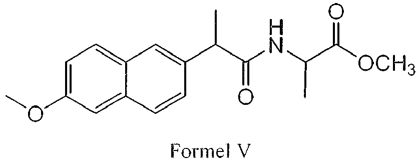 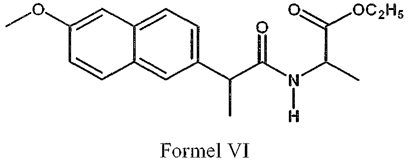 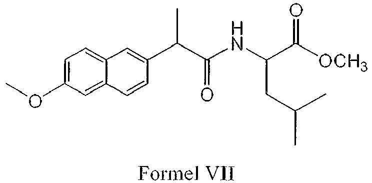 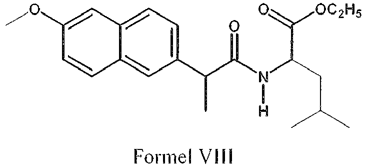 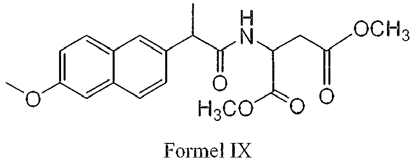 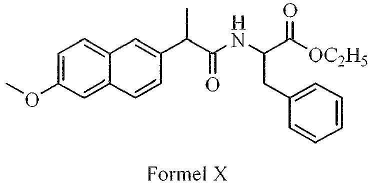 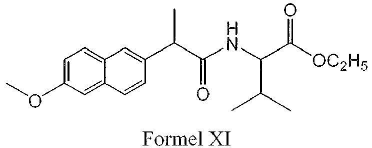 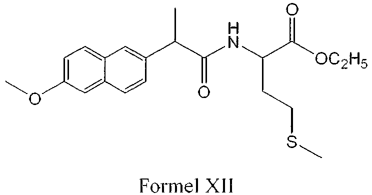 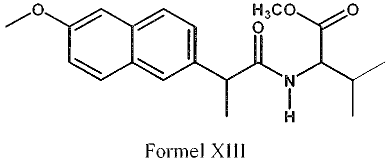 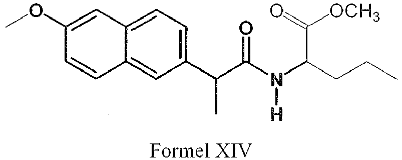 oder eine racemische Form oder ein Enantiomer der Substanz, zur Verwendung bei der Verhinderung oder Behandlung von Krebs.

2. Verbindung zur Verwendung nach Anspruch 1 mit der chemischen Struktur einer der Formeln III bis V, VII oder IX bis XII: 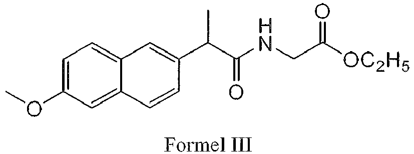 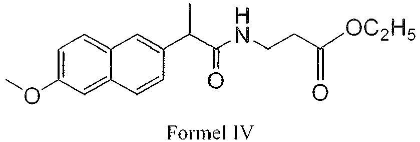 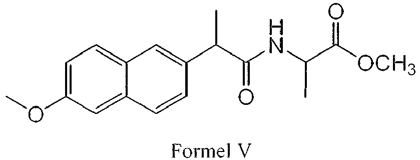 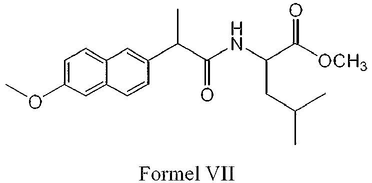 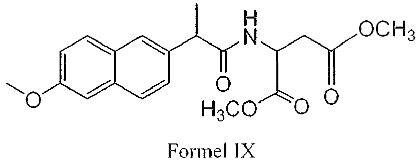 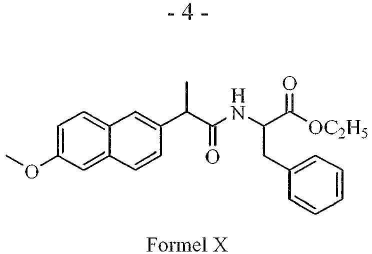 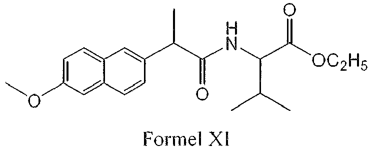 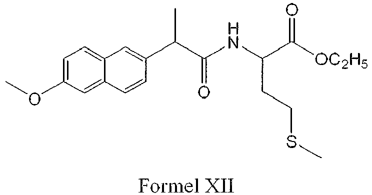 oder eine racemische Form oder ein Enantiomer der Substanz.

3. Verbindung zur Verwendung nach Anspruch 1 mit der chemischen Struktur nach einer der Formeln III, VII. X. XI oder XII.

4. Verbindung zur Verwendung nach einem der Ansprüche 1 bis 3, wobei der Krebs Prostatakrebs, Lungenkrebs, Kolorektalkrebs, Brustkrebs, Gehirnkrebs, Gebärmutterhalskrebs, Hodgkin-Lymphom, Nierenkrebs, Leukämie, Leberkrebs, Nicht-Hodgkin-Lymphom, Ovarialkrebs, Hautkrebs, Hodenkrebs, Schilddrüsenkrebs, Uteruskrebs oder Blasenkrebs ist, wobei bevorzugt der Krebs Kolorektalkrebs ist.

5. Pharmazeutische Zusammensetzung, umfassend eine Verbindung zur Verwendung nach einem der Ansprüche 1 bis 4 und einen pharmazeutisch akzeptablen Träger zur Verwendung als ein Medikament zur Behandlung von Krebs.

6. Pharmazeutische Zusammensetzung nach Anspruch 5 zur Verwendung als ein Medikament zur Behandlung von Krebs, wobei der Krebs bevorzugt Prostatakrebs, Lungenkrebs, Kolorektalkrebs, Brustkrebs, Gehirnkrebs, Gebärmutterhalskrebs, Hodgkin-Lymphom, Nierenkrebs, Leukämie, Leberkrebs, Nicht-Hodgkin-Lymphom, Ovarialkrebs, Hautkrebs, Hodenkrebs, Schilddrüsenkrebs, Uteruskrebs oder Blasenkrebs ist, wobei bevorzugter der Krebs Kolorektalkrebs ist.

7. Pharmazeutische Zusammensetzung zur Verwendung nach einem der Ansprüche 5 bis 6, wobei die pharmazeutische Zusammensetzung in einer Dosierungsform zu verabreichen ist, und die Dosierungsform eine Tablette, Pastille, Pille, Dragee, Kapsel, Flüssigkeit, Gel, Sirup, Schlamm, Suspension, Lösung oder Emulsion ist.

8. Pharmazeutische Zusammensetzung zur Verwendung nach einem der Ansprüche 5 bis 7 zur oralen, rektalen, transmukosalen, transdermalen, intestinalen, parenteralen, intramuskulären, intrathekalen, direkt intraventrikulären, intravenösen, intraperitonealen, intranasalen, intraokularen oder subkutanen Verabreichung.

9. Therapeutisches Mittel zur Verwendung in einem Verfahren zur Behandlung von Krebs, umfassend das Verabreichen an einen hierfür bedürftigen Patienten einer wirksamen Menge des therapeutischen Mittels, wobei das therapeutische Mittel eine Verwendung nach einem der Ansprüche 1 bis 4 oder eine pharmazeutische Zusammensetzung nach einem der Ansprüche 5 bis 8 umfasst.

10. Therapeutisches Mittel zur Verwendung nach Anspruch 9, wobei das therapeutische Mittel eine Verbindung nach Anspruch 1 bis 4 umfasst, und wobei der Krebs Prostatakrebs, Lungenkrebs, Kolorektalkrebs, Brustkrebs, Gehirnkrebs, Gebärmutterhalskrebs, Hodgkin-Lymphom, Nierenkrebs, Leukämie, Leberkrebs, Nicht-Hodgkin-Lymphom, Ovarialkrebs, Hautkrebs, Hodenkrebs, Schilddrüsenkrebs, Uteruskrebs oder Blasenkrebs ist, wobei bevorzugt der Krebs Kolorektalkrebs ist.

## Revendications

1. Composé ayant la structure chimique de l'une quelconque de la formule III à la formule XIV : 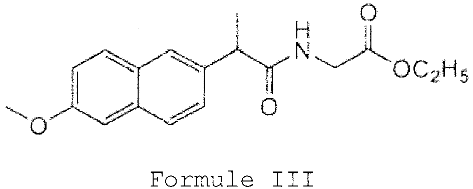 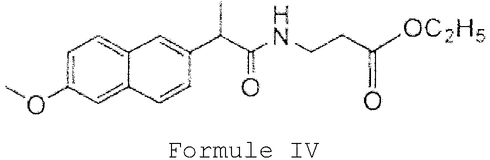 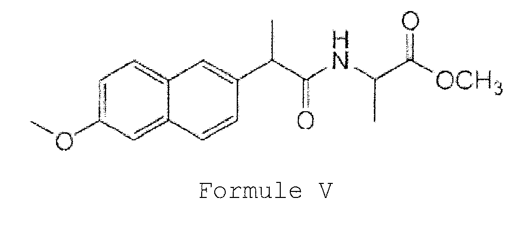 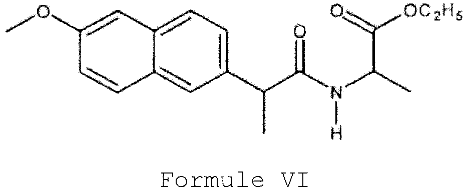 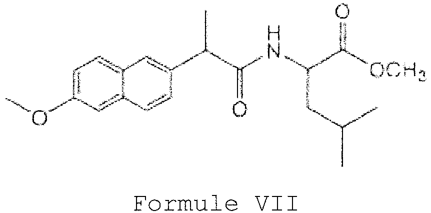 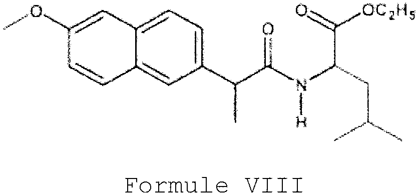 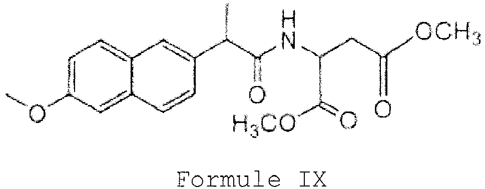 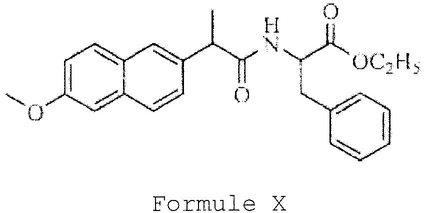 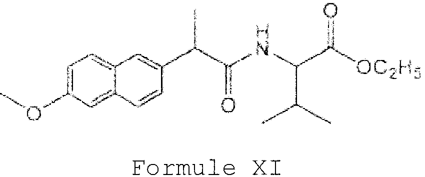 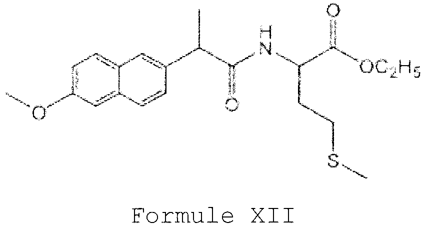 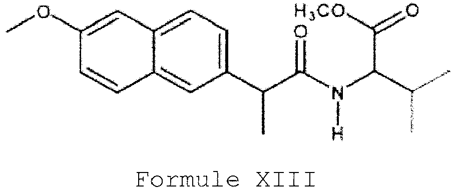 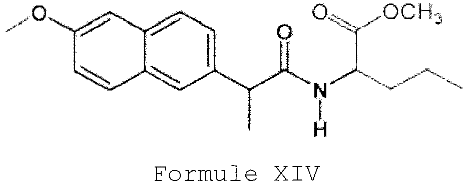 ou toute forme racémique ou tout énantiomère de la substance,
pour une utilisation dans la prévention ou le traitement du cancer.

2. Composé pour une utilisation selon la revendication 1, ayant la structure chimique de l'une quelconque de la formule III à V, VII, ou IX à XII : 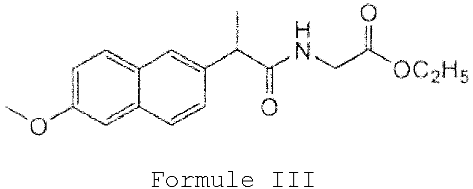 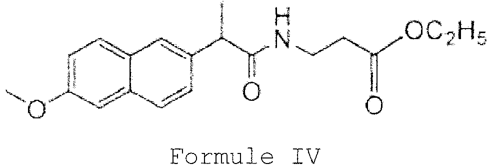 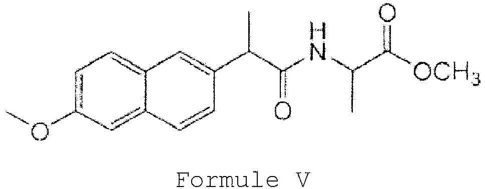 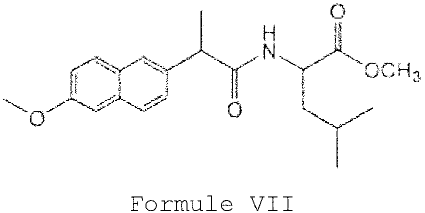 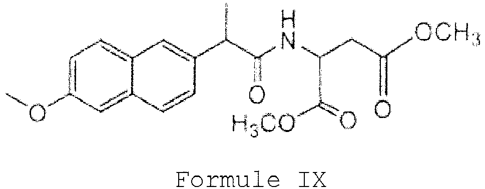 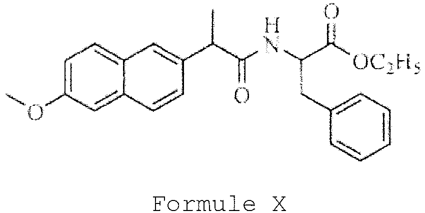 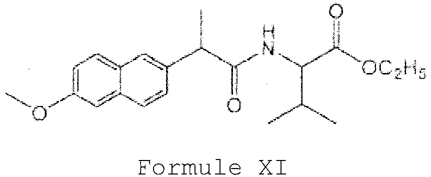 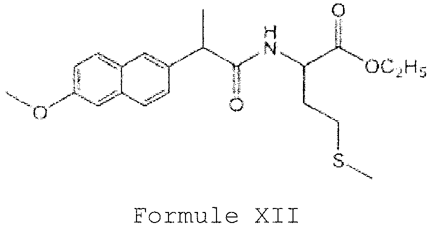 ou toute forme racémique ou tout énantiomère de la substance.

3. Composé pour une utilisation selon la revendication 1, ayant la structure chimique de l'une quelconque de la formule III, VII, X, XI, ou XII.

4. Composé pour une utilisation selon l'une quelconque des revendications 1 à 3, dans lequel le cancer est un cancer de la prostate, un cancer du poumon, un cancer colorectal, un cancer du sein, un cancer du cerveau, un cancer du col de l'utérus, un lymphome de Hodgkin, un cancer du rein, une leucémie, un cancer du foie, un lymphome non-hodgkinien, un cancer ovarien, un cancer de la peau, un cancer testiculaire, un cancer de la thyroïde, un cancer de l'utérus, ou un cancer de la vessie, dans lequel de préférence le cancer est un cancer colorectal.

5. Composition pharmaceutique comprenant un composé pour une utilisation selon l'une quelconque des revendications 1 à 4 et un support pharmaceutique acceptable pour une utilisation en tant que médicament pour le traitement du cancer.

6. Composition pharmaceutique selon la revendication 5 pour une utilisation en tant que médicament pour le traitement du cancer, dans laquelle le cancer est de préférence un cancer de la prostate, un cancer du poumon, un cancer colorectal, un cancer du sein, un cancer du cerveau, un cancer du col de l'utérus, un lymphome de Hodgkin, un cancer du rein, une leucémie, un cancer du foie, un lymphome non-hodgkinien, un cancer ovarien, un cancer de la peau, un cancer testiculaire, un cancer de la thyroïde, un cancer de l'utérus, ou un cancer de la vessie, dans laquelle de façon davantage préférée le cancer est un cancer colorectal.

7. Composition pharmaceutique pour une utilisation selon l'une quelconque des revendications 5 à 6, dans laquelle la composition pharmaceutique doit être administrée sous une forme galénique et la forme galénique est un comprimé, une pastille, une pilule, une dragée, une capsule, un liquide, un gel, un sirop, une suspension épaisse, une suspension, une solution ou une émulsion.

8. Composition pharmaceutique pour une utilisation selon l'une quelconque des revendications 5 à 7 pour une administration orale, rectale, transmucosale, transdermique, intestinale, parentérale, intramusculaire, intrathécale, intraventriculaire directe, intraveineuse, intrapéritonéale, intranasale, intraoculaire, ou sous-cutanée.

9. Agent thérapeutique pour une utilisation dans un procédé de traitement du cancer, comprenant l'administration à un sujet en ayant besoin d'une quantité efficace dudit agent thérapeutique, dans lequel l'agent thérapeutique comprend un composé selon l'une quelconque des revendications 1 à 4, ou une composition pharmaceutique selon l'une quelconque des revendications 5 à 8.

10. Agent thérapeutique pour une utilisation selon la revendication 9, dans lequel l'agent thérapeutique comprend un composé selon les revendications 1 à 4, et dans lequel le cancer est un cancer de la prostate, un cancer du poumon, un cancer colorectal, un cancer du sein, un cancer du cerveau, un cancer du col de l'utérus, un lymphome de Hodgkin, un cancer du rein, une leucémie, un cancer du foie, un lymphome non-hodgkinien, un cancer ovarien, un cancer de la peau, un cancer testiculaire, un cancer de la thyroïde, un cancer de l'utérus, ou un cancer de la vessie, dans lequel de préférence ledit cancer est un cancer colorectal.
